# FASCICULE DE BREVET EUROPEEN

(11) **EP 3 551 246 B1**
(45) Date de publication et mention de la délivrance du brevet: **21.10.2020**
(21) Numéro de dépôt: 17808106.3
(22) Date de dépôt: 20.11.2017
(51) Int. Cl.: A61M 1/10, A61M 1/12, A61B 17/34

(54) **DISPOSITIF D'ANCRAGE SANS SUTURE D'UNE POMPE CARDIAQUE**
VORRICHTUNG ZUR NAHTLOSEN VERANKERUNG FÜR HERZPUMPE
SUTURELESS ANCHORING DEVICE FOR HEART PUMP

(30) Priorité: 09.12.2016 FR 1662268
(43) Date de publication de la demande: 16.10.2019
(73) Titulaire: Fineheart, 33600 Pessac (FR)
(72) Inventeur: GARRIGUE, Stéphane, 33130 Begles (FR); MASCARELL, Arnaud, 37250 Montbazon (FR)
(74) Mandataire: Plasseraud IP
(86) Numéro de dépôt international: PCT/FR2017/053181
(87) Numéro de publication internationale: WO 2018/104605

(56) Documents cités:
- US-A1- 2007 106 315
- US-A1- 2014 378 772

## Description

### ARRIERE-PLAN DE L'INVENTION

### Domaine de l'invention

La présente invention concerne un dispositif de fixation sans suture sur une ouverture d'une paroi ventriculaire, notamment en vue de la pose d'une pompe cardiaque dans cette ouverture.

Elle concerne également un ensemble pour la pose/dépose d'une telle pompe cardiaque dans ce dispositif de fixation.

### Arrière-plan technologique

L'insuffisance cardiaque (IC), est un état pathologique dans lequel le cœur d'un patient présente une incapacité à fournir un débit sanguin nécessaire aux besoins métaboliques de l'organisme.

Il est connu pour traiter l'insuffisance cardiaque d'implanter un dispositif d'assistance ventriculaire (DAV), qui est une pompe cardiaque artificielle.

Cette pompe mécanique ne remplace pas le cœur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la survie et la qualité de vie des patients présentant une insuffisance cardiaque.

De nombreuses avancées ont été réalisées ces dernières années et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle.

On connaît des systèmes d'implantation de telles pompes dans un orifice d'une paroi ventriculaire.

Ces systèmes d'implantation comprennent généralement une portion tubulaire aux extrémités de laquelle sont placés, ou formés, des collerettes destinées à être plaquées chacune contre une face opposée de la paroi ventriculaire après introduction de la portion tubulaire dans un orifice réalisé dans cette paroi ventriculaire.

Ces collerettes permettent ainsi de maintenir en position cette portion tubulaire creuse, laquelle définit alors un conduit ouvert traversant la paroi ventriculaire.

A l'extrémité de cette portion tubulaire qui est placée à l'extérieur du cœur, est montée une pompe aspirative qui assure le renvoi du sang présent dans le ventricule vers la circulation corporelle.

US 2007/106315 A1 divulgue un système d'implantation de pompes cardiaques et représente l'art antérieur le plus proche de l'invention. Bien que représentant un progrès certain pour la qualité de vie d'un patient souffrant d'insuffisance cardiaque, de nombreux inconvénients sont encore constatés.

Notamment, on observe avec ces systèmes d'implantation de l'art antérieur des dommages qui sont causés à la paroi ventriculaire lors de leur installation, lesquels peuvent entraîner des déchirures localisées de cette paroi.

A titre purement illustratif, on connaît un tel système d'implantation dont une des brides vient être plaquée contre une face de la paroi ventriculaire par son déplacement le long de la surface externe de la portion tubulaire. On constate alors que l'opérateur ne pouvant contrôler de manière précise les efforts appliqués sur la paroi ventriculaire lors de la mise en aboutement de cette bride contre la face de la paroi, cette dernière vient typiquement écraser la paroi et abîmer celle-ci.

Par ailleurs, les dimensions des collerettes étant réduites pour autoriser leur passage au travers de l'orifice, la tenue mécanique du système d'implantation est limitée et n'autorise pas, par exemple, l'application d'efforts importants sur celui-ci une fois en place sur la paroi ventriculaire.

En outre, la pompe étant placée à l'extrémité de la portion tubulaire, extérieure au cœur, et le sang présent dans le ventricule passant dans le conduit ouvert, il existe un risque de perte de sang, notamment en raison des efforts appliqués sur la paroi ventriculaire.

Il existe donc un besoin pressant pour un dispositif de fixation sur un orifice d'une paroi ventriculaire dont le design original surmonte les inconvénients décrits ci-dessus.

### Objet de l'invention

La présente invention vise à pallier les inconvénients de l'art antérieur et à répondre aux contraintes ci-dessus énoncées en proposant un dispositif de fixation sans suture sur une ouverture d'une paroi ventriculaire, simple dans sa conception et dans son mode opératoire, fiable et préservant la paroi ventriculaire, c'est-à-dire non susceptible d'endommager cette paroi.

Un autre objet de la présente invention est un tel dispositif de fixation qui soit ajustable à des épaisseurs de paroi ventriculaire différentes.

Encore un objet de cette invention est un tel dispositif permettant d'y loger une pompe cardiaque et d'autoriser des opérations de maintenance sur cette pompe.

La présente invention vise également un dispositif de pose/dépose permettant aisément et sans risque de reflux sanguin, de poser et déposer une pompe cardiaque dans ce dispositif de fixation.

### BREVE DESCRIPTION DE L'INVENTION

A cet effet, l'invention concerne un dispositif de fixation sur une ouverture d'une paroi ventriculaire, comprenant
- un corps principal creux de forme générale cylindrique présentant une surface externe,
- une bague reçue à une première extrémité dudit corps creux, cette bague étant mobile le long d'au moins une partie de la surface externe du corps creux,
- une membrane tubulaire recouvrant la surface externe du corps creux en s'étendant entre ladite bague et l'extrémité opposée à la première extrémité du corps creux, dite seconde extrémité,
- une extrémité distale de cette membrane tubulaire, placée du côté de ladite seconde extrémité du corps creux, étant auto-expansible entre une première configuration dans laquelle elle présente une forme tubulaire, ou essentiellement tubulaire, et une seconde configuration dans laquelle elle définit une première bride s'étendant radialement, ou sensiblement radialement, à partir dudit corps creux, cette première bride étant destinée à venir en aboutement contre une face de ladite paroi ventriculaire,
- l'autre extrémité, dite proximale, de cette membrane tubulaire étant susceptible d'être déformée par le déplacement de ladite bague le long de la surface externe du corps creux de sorte à former une bride de retenue dont la position peut varier par rapport à ladite première bride de manière à s'adapter à des parois ventriculaires d'épaisseurs différentes,
- ladite bride de retenue étant destinée à venir en contact avec la face opposée de ladite paroi ventriculaire de sorte que lesdites brides et l'ensemble formé par la partie du corps creux et la portion de la membrane tubulaire la recouvrant, traversant ladite paroi ventriculaire au travers de ladite ouverture, bloquent en position ledit dispositif de fixation dans ladite ouverture.

Cette bague, dite de pressage, présente une forme adaptée pour s'engager sur la surface externe dudit corps creux. Elle est placée à, ou proche de, ladite première extrémité du corps creux.

De manière avantageuse, cette bague permet de mettre en compression la membrane tubulaire placée sur la surface externe dudit corps creux. Le degré de mise en compression, ou pressage, de la membrane tubulaire est contrôlé par l'opérateur en déplaçant plus ou moins la bague le long de la surface externe du corps creux. On contrôle ainsi avantageusement les efforts appliqués sur la paroi ventriculaire.

Elle présente à cet effet sur son bord destiné à venir en contact avec l'extrémité proximale de ladite membrane tubulaire, une surface annulaire de pressage de ladite membrane tubulaire. Cette surface annulaire peut comporter un logement pour recevoir l'extrémité proximale de la membrane tubulaire.

L'extrémité proximale de la membrane tubulaire est donc susceptible d'être déformée en s'évasant vers l'extérieur de manière à former une bride de retenue écartée de la première bride. Cette bride élargie radialement présente avantageusement une surface planaire ou sensiblement planaire destinée à faire face à la paroi ventriculaire.

Le corps creux présente une dimension longitudinale supérieure à celle de l'ouverture s'étendant entre les faces opposées de la paroi ventriculaire et un diamètre égal ou supérieur à celui de l'ouverture dans la paroi ventriculaire.

Ainsi, et de manière avantageuse, le dispositif de fixation sur une ouverture d'une paroi ventriculaire assure une étanchéité après pose sans réalisation d'aucune suture, contrairement aux dispositifs d'ancrage de l'état de l'art. En ce sens, il s'agit d'un dispositif de fixation sur une ponction ventriculaire d'un diamètre supérieure à 20 mm, ne nécessitant aucun point de suture.

Dans différents modes de réalisation particuliers de ce dispositif, chacun ayant ses avantages particuliers et susceptibles de nombreuses combinaisons techniques possibles:
- l'extrémité proximale de la membrane tubulaire est configurée pour être progressivement déformée lors du déplacement de la bague de manière à former une bride de retenue dont la position peut varier dans une plage prédéterminée de distances à partir de la première bride.
- ladite bague comporte sur son pourtour au moins un doigt d'accouplement destiné à coopérer avec des crans placés sur la surface externe dudit corps creux. On obtient ainsi un déplacement discret, ou ponctuel, de la bague le long de la surface externe du corps creux.
Alternativement, la surface externe dudit corps creux comprenant un filetage sur au moins une partie de sa surface externe, la surface interne de ladite bague comporte un filetage configuré pour coopérer avec ledit filetage placé sur la surface externe du corps creux afin de permettre le déplacement de ladite bague.
- ladite bague comporte un doigt anti-retour pour empêcher un éventuel desserrement de la bague après formation de la bride de retenue.
- la surface externe du corps creux comporte une butée pour stopper le déplacement de ladite bague,
- ladite bague et ledit corps creux sont réalisés dans des matériaux rigides et inertes pour le corps humain,
- ladite membrane tubulaire est réalisée en nitinol, dans un alliage de nitinol ou encore en polyuréthane expansible,
- le dispositif est configuré pour former une bride de retenue ayant un diamètre externe strictement supérieur 3/2 D où D est le diamètre de l'ouverture dans la paroi ventriculaire.
De préférence, la bride de retenue ainsi formée présente un diamètre externe compris entre 3/2 D et 3 D.
A titre purement illustratif, cette bride de retenue présente un diamètre de 40 mm.
Cette grande dimension de la bride de retenue destinée à être ainsi formée, renforce l'appui du dispositif sur la paroi ventriculaire et assure, de ce fait, une meilleure tenue en position, ou stabilisation, du dispositif de fixation sur cette paroi ventriculaire notamment lors d'une intervention sur la pompe cardiaque propulsive destinée à être reçue dans le canal de ce corps principal.
Par ailleurs, d'éventuelles contraintes sont réparties sur une plus grande surface de la paroi ventriculaire de sorte que le risque d'endommager cette paroi est réduit, voire supprimé.
- la paroi interne du corps creux délimitant un canal s'étendant entre les première et seconde extrémités du corps creux, ladite paroi interne comporte des moyens pour l'assemblage étanche du corps d'une pompe cardiaque et de ladite paroi, ledit corps de pompe étant ainsi reçu au moins en partie dans ledit canal.
Le diamètre du canal est égal ou sensiblement égal au diamètre externe du corps de pompe pour loger cette dernière dans ledit canal.
De préférence, le corps de la pompe destinée à être logée dans le canal délimité par la paroi interne du corps creux comportant un filetage sur sa surface externe, cette paroi interne comprend un filetage destiné à coopérer avec le filetage du corps de pompe.
- ladite pompe cardiaque est un dispositif d'assistance ventriculaire (DAV) implantable.

De préférence, il s'agit d'une pompe cardiaque propulsive.

Cette pompe cardiaque étant ancrée à la paroi du cœur au moyen du dispositif de fixation, le patient peut dès lors se déplacer de manière active sans aucun risque.

La première extrémité du corps principal creux comporte une ouverture en communication avec le canal délimité par la paroi intérieure du corps creux. De manière avantageuse, une ou plusieurs liaisons filaires telles que des câbles, peuvent passer au travers de cette ouverture pour relier une pompe cardiaque artificielle logée dans ce canal à une unité de gestion de cette pompe. Des signaux de commande de cette pompe peuvent ainsi être envoyés à la pompe artificielle.

Cette unité de gestion peut également comprendre un émetteur-récepteur sans fil pour transmettre automatiquement des données telles que des informations sur le rythme cardiaque ou encore l'état de la source d'alimentation implantée, en vue d'un suivi de télémédecine.

La transmission des données peut être réalisée vers un terminal externe portatif au moyen de signaux de communication sans fil à courte portée, par exemple basés sur un protocole bluetooth ou Zigbee, .... Ce terminal externe peut comporter un moyen de communication mettant en œuvre un réseau d'accès cellulaire et/ou un réseau internet pour transmettre ces données vers par exemple un cardiologue. Le réseau d'accès cellulaire peut être de plusieurs types (2G, 3G, 4G), chaque type de réseau étant accessible selon plusieurs technologies d'accès cellulaires (2G : EDGE, GPRS, 3G : UMTS, HSDPA, HSUPA, HSPA, HSPA+, 4G : LTE). Le réseau internet est par exemple un réseau comportant des points d'accès non cellulaires sans fil tel qu'un réseau WLAN, par exemple Wi-Fi ou WiMAX ou encore d'un réseau Li-Fi. Ce terminal externe peut présenter un dispositif d'affichage pour permettre à l'utilisateur de lire des messages ou de choisir des options dans un menu.

De préférence, cette unité centrale comprend une ou plusieurs entrées pour recevoir un ou plusieurs signaux dont chacun est lié à une vibration mécanique audible ou inaudible liée à l'activité mécanique du cœur, ladite unité centrale comprenant un premier sous-ensemble d'instructions logicielles dudit ensemble d'instructions logicielles qui lorsqu'elles sont exécutées par ledit processeur, sont configurées pour définir une fenêtre temporelle de mesure dudit ou desdits signaux, pour analyser chaque signal ainsi reçu à l'entrée de ladite unité centrale durant cette fenêtre temporelle afin de déterminer un ou plusieurs paramètres du signal correspondant, pour comparer le ou les paramètres de chaque signal ainsi déterminés avec une ou plusieurs données préalablement enregistrées dans une unité de stockage de ladite unité centrale afin d'identifier le signal correspondant à la fermeture de la vanne mitrale ainsi que l'instant t₁ correspondant à la fermeture de ladite valve mitrale.

La présente invention concerne également une unité d'assistance ventriculaire comprenant une pompe cardiaque propulsive et un dispositif de fixation sur une ouverture dans une paroi ventriculaire, tel que décrit précédemment.

De manière avantageuse, cette pompe cardiaque propulsive est logée à l'intérieur du canal délimité par la paroi interne du corps principal creux. De préférence, le corps de pompe et la paroi interne du corps principal creux sont assemblés de manière étanche pour éviter tout reflux de sang.

La présente invention concerne encore un ensemble pour la pose/dépose d'une pompe cardiaque sur un dispositif de fixation tel que décrit précédemment, ledit ensemble comprenant un élément de guidage présentant une extrémité distale, une extrémité proximale et une lumière s'étendant entre et débouchant auxdites extrémités distale et proximale, ladite pompe cardiaque ayant un corps de pompe.

Selon l'invention,
- ledit corps de pompe comprenant une empreinte,
- ledit ensemble comprend un organe de préhension apte à coulisser dans ladite lumière, ledit organe de préhension comportant à son extrémité libre, une partie complémentaire de ladite empreinte de manière à assurer l'engagement de cette extrémité libre et du corps de pompe pour la préhension et le déplacement de ladite pompe cardiaque.

De préférence, ladite empreinte étant un creux, ou une saillie, ménagés sur ou dans le corps de pompe, ladite partie complémentaire est une tête de forme conjuguée audit creux, respectivement un creux de forme conjuguée à ladite saillie, pour l'assemblage de ladite extrémité libre audit corps de pompe.

De manière avantageuse, l'extrémité libre dudit organe de préhension comporte un profil polygonal complémentaire d'un profil en creux placé à une extrémité du corps de pompe cardiaque de sorte que cette extrémité libre puisse s'insérer dans ledit profil en creux.

Avantageusement, ledit corps de pompe comprenant un filetage sur une partie de sa surface externe, lequel est distinct de ladite empreinte, ledit organe de préhension est un corps allongé de forme générale cylindrique, pour être manœuvrable en rotation dans ladite lumière afin de visser/dévisser ledit corps de pompe sur un filetage complémentaire porté par la paroi intérieure du corps creux. L'organe de préhension peut ainsi être un tube creux ou une tige, dont les extrémités libres sont des parties complémentaires de l'empreinte d'un corps de pompe pour autoriser sa manipulation.

De préférence, ledit corps de pompe comportant au moins deux empreintes, ladite extrémité libre dudit organe de préhension comprend pour chaque empreinte, une partie d'assemblage complémentaire de l'empreinte correspondante.

Cet ensemble peut comporter un élément d'étanchéité placé à l'intérieur de ladite lumière, et de préférence dans sa partie distale, cet élément d'étanchéité étant ouvert lorsque l'extrémité libre dudit organe de préhension est pressée contre celui-ci, afin de libérer le passage audit organe de préhension au travers de cet élément d'étanchéité.

De préférence, cet élément d'étanchéité comprenant une vanne clapet dont le siège est incliné à 45° pour faciliter le passage du dispositif médical, tout en empêchant le passage du sang dans l'autre direction lorsque cette vanne clapet est dans sa position d'obturation. Cette vanne clapet définit ainsi une vanne anti-retour.

De manière préférentielle, l'extrémité distale dudit élément de guidage comporte un dispositif de stabilisation d'une partie du cœur, ledit dispositif de stabilisation comportant une partie évasée comprenant, de préférence à son extrémité de plus large diamètre, une rainure, laquelle présente au moins un orifice d'aspiration pour créer un vide dans ladite rainure lorsqu'une portion de paroi ventriculaire est en contact avec ledit dispositif de stabilisation.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels :
- la Figure 1 représente schématiquement un dispositif d'ancrage sans suture pour une pompe cardiaque propulsive selon un mode de réalisation particulier de l'invention ; l'extrémité distale de la membrane tubulaire étant dans sa première configuration stable, et la bague n'ayant pas encore été déplacée le long de la surface externe du tube pour mettre en compression l'extrémité proximale de la membrane tubulaire ;
- la Figure 2 montre le dispositif d'ancrage sans suture de la Fig. 1 dans un état activé, dans lequel l'extrémité distale de la membrane tubulaire est dans sa deuxième configuration stable et l'extrémité proximale de la membrane tubulaire a été déformée pour définir une bride de retenue, les faces opposées d'une paroi ventriculaire (PV) ont été schématisées dans la partie supérieure du dispositif pour illustrer la mise en contact des brides sur ces faces opposées, l'espace extra-cardiaque (EC) étant également rappelé ;
- la Figure 3 est une représentation schématique partielle et en coupe du dispositif d'ancrage illustré à la Fig. 1 ;
- la Figure 4 est une représentation schématique partielle et en coupe du dispositif d'ancrage illustré à la Fig. 2 ;
- la Figure 5 est une vue en perspective de la bague du dispositif d'ancrage de la Fig. 1 montrant le clapet anti-retour de la bague ;
- la Figure 6 est une vue en coupe longitudinale d'un ensemble de pose/dépose d'une pompe cardiaque reliée à l'apex d'un cœur battant, selon un mode de réalisation particulier de l'invention, cet ensemble étant montré dans une première configuration dans laquelle il comprend un outil de perforation et d'ablation d'une partie de la paroi ventriculaire pour réaliser une ouverture dans ladite paroi ;
- la Figure 7 est une vue en coupe longitudinale de l'ensemble de pose/dépose de la Fig. 6, selon une autre configuration dans laquelle il comporte un organe de préhension relié et supportant une pompe cardiaque, laquelle est engagée dans un dispositif d'ancrage fixé dans l'ouverture d'une paroi ventriculaire, une partie de la pompe cardiaque étant intraventriculaire ;
- la Figure 8 représente de manière partielle et schématique, l'ensemble de pose/dépose d'une pompe cardiaque de la Fig. 7, la pompe cardiaque étant montée à l'intérieur du ventricule ;

### DESCRIPTION DETAILLEE DE MODE DE REALISATION DE L'INVENTION

Tout d'abord, on note que les figures ne sont pas à l'échelle.

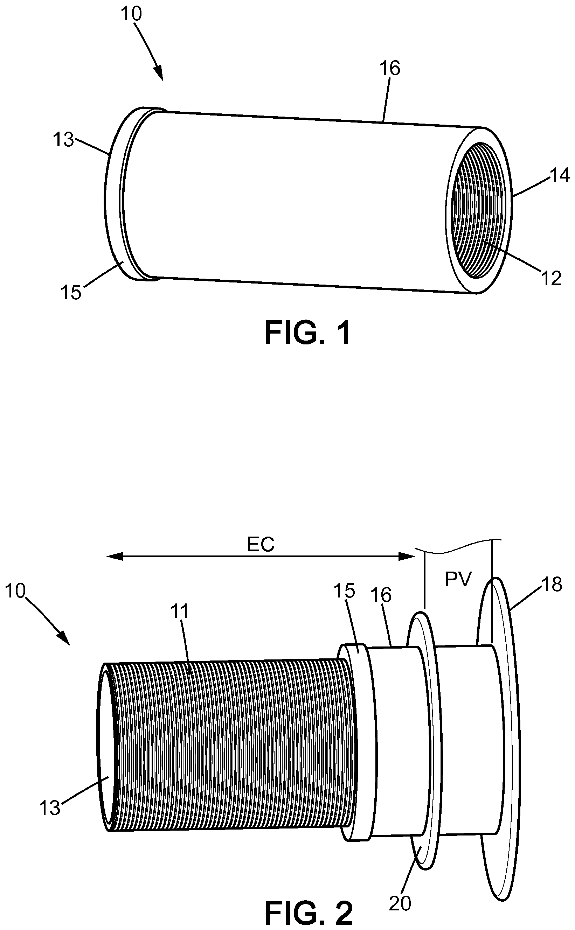

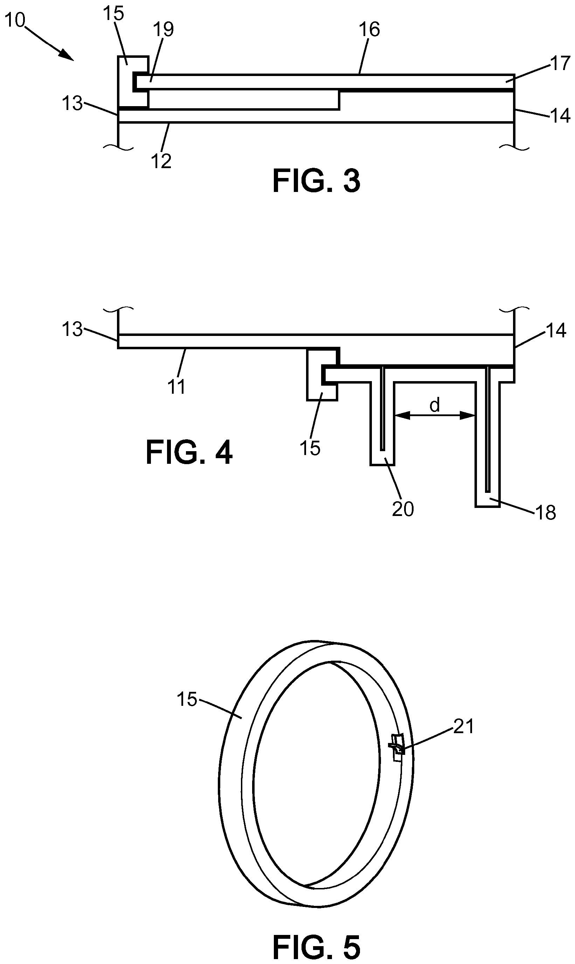

Les Figures 1 à 5 montrent de manière schématique un dispositif d'ancrage 10 sans suture d'une pompe cardiaque propulsive dans une ouverture d'une paroi ventriculaire, selon un mode de réalisation particulier de la présente invention.

Ce dispositif d'ancrage 10 comporte un tube présentant une surface externe 11 et une surface intérieure 12 délimitant un canal s'étendant entre une première extrémité 13 et une seconde extrémité 14, ouvertes de ce tube.

Les surfaces externe 11 et intérieure 12 de ce tube 10 présentent un filetage, la surface externe 11 du tube comprenant du côté de sa seconde extrémité 14, une portion de paroi présentant une surépaisseur, laquelle est non filetée et reliée par un épaulement au reste de la surface externe 11 du tube filetée. Cet épaulement définit ainsi une butée pour limiter le déplacement d'une bague 15 de pressage reçue à la première extrémité 13 du tube.

La surface interne de cette bague 15 comporte un filetage (non représenté) destiné à coopérer avec le filetage de la surface externe 11 du tube pour autoriser un déplacement continu de cette bague 15 le long d'une partie de la surface externe 11 de ce tube.

Entre cette bague 15 et la seconde extrémité 14 du tube est placée une membrane tubulaire 16 recouvrant la surface externe 11 du tube.

La bague 15 comporte sur sa face destinée à presser la membrane tubulaire 16, un logement tel qu'une rainure, pour recevoir l'extrémité libre correspondante de la membrane tubulaire 16. De manière avantageuse, cette extrémité de la membrane tubulaire 16, encore appelée extrémité proximale, est non solidarisée dans son logement pour éviter les torsions de la membrane.

Une extrémité 17 distale de cette membrane tubulaire 16, placée du côté de la seconde extrémité 14 du tube, est auto-expansible entre une première configuration stable dans laquelle elle présente une forme tubulaire, et une seconde configuration stable dans laquelle elle définit une première bride 18 s'étendant radialement à partir dudit tube.

La première configuration stable de la membrane permet avantageusement une introduction aisée de la seconde extrémité 14 du tube au travers de l'ouverture de la paroi ventriculaire.

La seconde configuration stable de la membrane tubulaire 16 permet de générer une première bride 18 de sorte que celle-ci vienne en aboutement, ou encore soit plaquée, contre une face de la paroi ventriculaire lorsque le tube a été introduit au travers de l'ouverture de cette paroi ventriculaire.

Le passage de la première configuration stable à la seconde configuration stable est obtenu par une augmentation de la température de la membrane tubulaire 16, par exemple, en exposant celle-ci à la température du corps humain, la membrane tubulaire 16 étant réalisée en nitinol ou en polyuréthane expansé-matériel à mémoire de forme.

L'extrémité opposée, dite proximale 19, de cette membrane tubulaire 16 est susceptible d'être déformée progressivement par le déplacement de la bague 15 le long de la surface externe 11 du tube de manière à former une seconde bride 20 de retenue dont la position peut varier dans une plage prédéterminée de distances d à partir de la première bride 18 définie par l'extrémité distale 17 de la membrane tubulaire 16 dans sa seconde configuration stable. Cette distance d permet de comprimer la paroi ventriculaire afin d'assurer l'étanchéité du dispositif et un maintien fiable et durable.

Il est ainsi possible d'adapter le dispositif d'ancrage 10 à des parois ventriculaires d'épaisseurs différentes. L'épaulement déterminé par le profil de la surface 11 externe du tube permet avantageusement de définir une limite supérieure sur la mise en compression de la membrane tubulaire 16 et ainsi de limiter les efforts appliqués sur la paroi ventriculaire.

De manière avantageuse, cette bague 15 comporte un doigt 21 anti-retour.

Ce doigt 21 anti-retour permet d'empêcher un éventuel desserrement de la bague 15 après formation de la seconde bride 20 de retenue, lequel serait susceptible d'entraîner un relâchement de, ou une libération partielle des contraintes dans, la membrane tubulaire 16, ayant pour conséquence, un affaissement de la seconde bride 20 de retenue. Une telle déformation de la seconde bride 20 de retenue serait susceptible d'entraîner une moins bonne étanchéité de la liaison entre le dispositif d'ancrage et la paroi ventriculaire susceptible de résulter en des fuites de sang.

La bague 15 et le tube sont réalisés dans des matériaux rigides et inertes, c'est-à-dire biocompatibles avec l'organisme humain. Ils sont, par exemple, réalisés en PEEK (polyétheréthercétone), en céramique ou en titane. Ces éléments peuvent être imprimés, c'est-à-dire qu'ils sont alors formés par un procédé d'impression tridimensionnelle.

Une fois, ce dispositif d'ancrage 10 solidarisé à la paroi ventriculaire, il est possible d'introduire dans le canal délimité par la surface intérieure 12 du tube, une pompe cardiaque propulsive.

Cette pompe (non représentée) est avantageusement destinée à être reçue dans le canal du tube de sorte qu'elle ne forme pas saillie à l'extérieur du tube à l'extérieur du cœur.

En outre, l'assemblage du corps de pompe et de la surface 12 intérieure du tube est étanche pour empêcher tout reflux sanguin par ce canal. Un tel assemblage est avantageusement réalisé ici par vissage du corps de pompe sur le filetage porté par la surface intérieure 12 du tube.

Pour cela, le corps de pompe présente sur au moins une partie de sa surface externe 11 un filetage destiné à coopérer avec le filetage porté par la surface 12 intérieure du tube.

Le vissage de la pompe au sein de la surface intérieure 12 du tube permet également de régler, ou ajuster, le positionnement de cette pompe vis-à-vis de la valve aortique et, par conséquent, d'optimiser la position de cette pompe cardiaque pour atteindre un meilleur rendement hémodynamique. De manière avantageuse, on obtient ainsi un débit cardiaque optimisé, lequel est permis par la possibilité de réaliser un déplacement continu, et par conséquent, extrêmement précis, du corps de pompe le long du filetage formé à la surface intérieure 12 du tube.

Ainsi, et de manière plus générale, la présente invention concerne également un procédé visant à optimiser le rendement hémodynamique, dans lequel on ajuste la position d'une pompe cardiaque implantée sur une paroi ventriculaire par rapport à la valve aortique du patient. De préférence, cet ajustement est obtenu par un déplacement de la pompe cardiaque par rapport à cette valve aortique. Et encore mieux, cet ajustement est réalisé par vissage/dévissage du corps de la pompe le long d'une surface filetée interne, ou encore sur le côté intérieur fileté, d'une paroi tubulaire d'un dispositif de fixation, ou d'ancrage, fixé dans une ouverture d'une paroi ventriculaire, le corps de pompe comprenant, à cet effet, sur sa surface externe un filetage complémentaire du filetage du côté intérieur fileté de la paroi tubulaire et destiné à coopérer avec ce dernier. Un tel ajustement en position est avantageusement très précis en raison du déplacement continu de la pompe cardiaque, autorisé par ces filetages.

On cherche ainsi à positionner l'extrémité de la pompe cardiaque pour diriger le sang propulsé par cette pompe vers la valve aortique du patient. De préférence, on cherche à placer cette extrémité de la pompe cardiaque à une distance comprise entre 10 mm et 20 mm de la valve aortique.

Afin de limiter le déplacement de la pompe cardiaque par vissage de cette dernière le long de la surface intérieure 12 du tube, cette surface intérieure 12 du tube peut comporter au moins une butée. On peut ainsi contrôler l'avancée intraventriculaire de la pompe cardiaque.

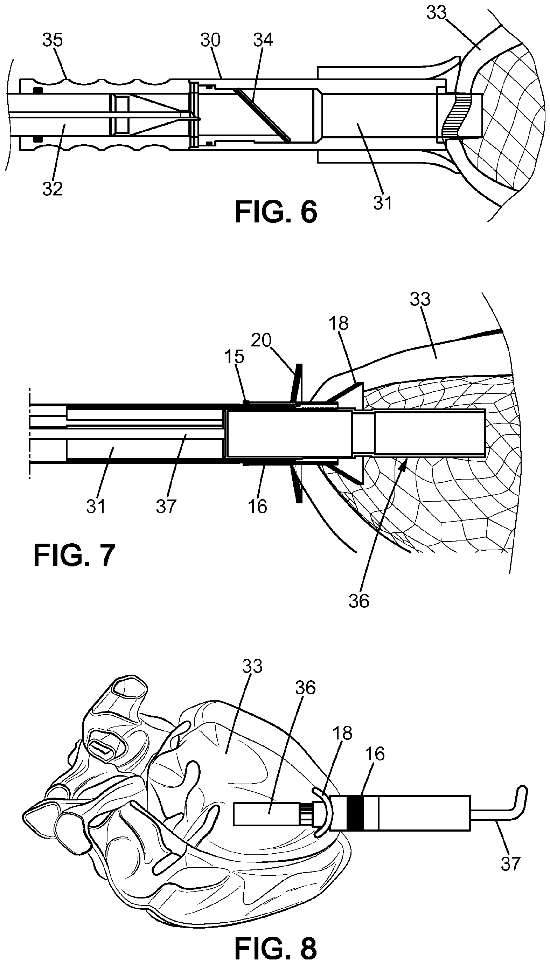

Les Figures 6 à 8 illustrent un système pour l'introduction d'un dispositif médical tel qu'une aiguille, un dispositif d'ancrage 10 sur un orifice d'une paroi ventriculaire tel que décrit ci-dessus, une pompe cardiaque 36 ou encore un organe de préhension de cette pompe, selon un mode de réalisation particulier de la présente invention.

Ce système pour l'introduction d'un dispositif médical comprend un corps 30 principal définissant un canal 31 intérieur longitudinal, ou lumière, pour recevoir ce dispositif médical 32, ce dispositif médical étant mobile en translation dans ce canal 31 intérieur de sorte qu'une partie de celui-ci peut être placée en saillie de ce corps 30 principal pour son introduction dans une paroi ventriculaire 33 ou dans le canal délimité par la paroi intérieure 12 d'un manchon fixé dans l'ouverture de la paroi ventriculaire 33, tel que celui d'un dispositif d'ancrage décrit plus haut.

Ce corps 30 principal comprend également un élément d'étanchéité 34 placé à l'intérieur de ce canal de telle sorte que cet élément d'étanchéité 34 peut être ouvert lorsque l'extrémité du dispositif médical est pressée contre celui-ci, afin de libérer le passage au dispositif médical 32 à travers cet élément d'étanchéité 34.

Cet élément d'étanchéité 34 comprend ici une vanne clapet dont le siège est incliné à 45° pour faciliter le passage du dispositif médical 32, tout en empêchant le passage du sang dans l'autre direction lorsque cette vanne clapet est dans sa position d'obturation. Cette vanne clapet forme ainsi une vanne anti-retour.

L'extrémité proximale du corps 30 principal comporte une poignée 35 de préhension et l'extrémité distale du corps principal comporte avantageusement une valve de purge (non représentée) afin d'éliminer toute présence d'air dans la partie du canal intérieur placée en aval de l'élément d'étanchéité 34.

Afin de manipuler la pompe cardiaque 36 pour son implantation dans, ou son démontage, du canal délimité par la paroi intérieure 12 du tube, ce système comporte un organe 37 de préhension apte à coulisser dans le canal 31 intérieur, cet organe 37 de préhension présentant à son extrémité libre, une partie complémentaire d'une empreinte portée par le corps de pompe.

Il est ainsi possible d'assurer l'engagement de l'extrémité libre de cet organe 37 de préhension et de l'empreinte du corps de pompe pour la préhension et la manipulation de la pompe cardiaque 36.

A titre purement illustratif, le corps de pompe présentant une empreinte polygonale creuse telle qu'à six pans ménagés dans un creux du corps de pompe, l'extrémité libre de l'organe 37 de préhension présente une forme complémentaire telle qu'une forme mâle hexagonale.

Cet organe 37 de préhension se présente avantageusement sous la forme d'une tige que l'opérateur peut manipuler par son extrémité proximale de manière à entraîner en rotation celle-ci afin de visser, ou dévisser, le corps de pompe dans son logement défini par le canal délimité par la paroi intérieure 12 du tube.

## Revendications

1. Dispositif de fixation sur une ouverture d'une paroi ventriculaire, comprenant
- un corps principal creux de forme générale cylindrique présentant une surface (11) externe,
- une bague (15) reçue à une première extrémité dudit corps creux, cette bague (15) étant mobile le long d'au moins une partie de la surface (11) externe du corps creux,
- une membrane (16) tubulaire recouvrant la surface (11) externe du corps creux en s'étendant entre ladite bague (15) et l'extrémité opposée à la première extrémité du corps creux, dite seconde extrémité,
- une extrémité (17) distale de cette membrane (16) tubulaire, placée du côté de ladite seconde extrémité du corps creux, étant auto-expansible entre une première configuration dans laquelle elle présente une forme tubulaire, et une seconde configuration dans laquelle elle définit une première (18) bride s'étendant radialement, ou sensiblement radialement, à partir dudit corps creux, cette première (18) bride étant destinée à venir en aboutement contre une face de ladite paroi ventriculaire,
- l'autre extrémité, dite proximale, de cette membrane (16) tubulaire étant susceptible d'être déformée par le déplacement de ladite bague (15) le long de la surface (11) externe du corps creux de sorte à former une bride (20) de retenue dont la position peut varier par rapport à ladite première (18) bride de manière à s'adapter à des parois ventriculaires d'épaisseurs différentes,
- ladite bride (20) de retenue étant destinée à venir en contact avec la face opposée de ladite paroi ventriculaire de sorte que lesdites brides et l'ensemble formé par la partie du corps creux et la portion de la membrane (16) tubulaire la recouvrant, traversant ladite paroi ventriculaire au travers de ladite ouverture, bloquent en position ledit dispositif de fixation dans ladite ouverture.

2. Dispositif selon la revendication 1, **caractérisé en ce que** l'extrémité proximale de la membrane (16) tubulaire est configurée pour être progressivement déformée lors du déplacement de la bague (15) de manière à former une bride (20) de retenue dont la position peut varier dans une plage prédéterminée de distances à partir de la première (18) bride.

3. Dispositif selon la revendication 1 ou 2, **caractérisé en ce que** ladite bague (15) comporte sur son pourtour au moins un doigt d'accouplement destiné à coopérer avec des crans placés sur la surface (11) externe dudit corps creux.

4. Dispositif selon la revendication 1 ou 2, **caractérisé en ce que** la surface (11) externe dudit corps creux comprenant un filetage sur au moins une partie de sa surface (11) externe, la surface interne de ladite bague (15) comporte un filetage configuré pour coopérer avec ledit filetage placé sur la surface (11) externe du corps creux afin de permettre le déplacement de ladite bague (15).

5. Dispositif selon l'une quelconque des revendications 1 à 4, **caractérisé en ce que** ladite bague (15) comporte un doigt (21) anti-retour pour empêcher un éventuel desserrement de la bague (15) après formation de la bride (20) de retenue.

6. Dispositif selon l'une quelconque des revendications 1 à 5, **caractérisé en ce que** la surface (11) externe du corps creux comporte une butée pour stopper le déplacement de ladite bague (15).

7. Dispositif selon l'une quelconque des revendications 1 à 6, **caractérisé en ce que** ladite bague (15) et ledit corps creux sont réalisés dans des matériaux rigides et inertes pour le corps humain.

8. Dispositif selon l'une quelconque des revendications 1 à 7, **caractérisé en ce que** ladite membrane (16) tubulaire est réalisée en nitinol, dans un alliage de nitinol ou encore en polyuréthane expansible.

9. Dispositif selon l'une quelconque des revendications 1 à 8, **caractérisé en ce qu'**il est configuré pour former une bride (20) de retenue ayant un diamètre externe strictement supérieur 3/2 D où D est le diamètre de l'ouverture dans la paroi ventriculaire.

10. Dispositif selon l'une quelconque des revendications 1 à 9, **caractérisé en ce que** la paroi (12) interne du corps creux délimitant un canal s'étendant entre les première et seconde extrémités du corps creux, ladite paroi (12) interne comporte des moyens pour l'assemblage étanche du corps d'une pompe cardiaque et de ladite paroi, ledit corps de pompe étant ainsi reçu au moins en partie dans ledit canal.

11. Dispositif selon la revendication 10, **caractérisé en ce que** le corps de la pompe destinée à être logée dans le canal délimité par la paroi (12) interne du corps creux comportant un filetage sur sa surface (11) externe, cette paroi (12) interne comprend un filetage destiné à coopérer avec le filetage du corps de pompe.

12. Unité d'assistance ventriculaire comprenant une pompe cardiaque propulsive et un dispositif de fixation selon l'une quelconque des revendications 1 à 11.

## Patentansprüche

1. Vorrichtung zur Fixierung an einer Öffnung einer Ventrikelwand, umfassend
- einen allgemein zylindrischen, hohlen Hauptkörper, der eine Außenfläche (11) aufweist,
- einen an einem ersten Ende des hohlen Körpers aufgenommenen Ring (15), wobei der Ring (15) entlang mindestens einem Teil der Außenfläche (11) des hohlen Körpers beweglich ist,
- eine röhrenförmige Membran (16), die die Außenfläche (11) des hohlen Körpers abdeckt, indem sie sich zwischen dem Ring (15) und dem Ende erstreckt, das dem ersten Ende des hohlen Körpers gegenüberliegt, und zwar dem zweiten Ende,
- wobei ein distales Ende (17) dieser röhrenförmigen Membran (16), das an der Seite des zweiten Endes des hohlen Körpers platziert ist, zwischen einer ersten Konfiguration, in der es röhrenförmig ist, und einer zweiten Konfiguration, in der es einen ersten Flansch (18) definiert, der sich radial oder im Wesentlichen radial von dem hohlen Körper erstreckt, selbstexpandierbar ist, wobei der erste Flansch (18) dazu bestimmt ist, an einer Fläche der Ventrikelwand zur Anlage zu kommen,
- wobei das andere Ende, das proximale Ende, dieser röhrenförmigen Membran (16) durch das Verschieben des Rings (15) entlang der Außenfläche (11) des hohlen Körpers deformiert werden kann, so dass ein Halteflansch (20) gebildet wird, dessen Position bezüglich des ersten Flanschs (18) zur Anpassung an Ventrikelwände mit unterschiedlicher Dicke variieren kann,
- wobei der Halteflansch (20) dazu bestimmt ist, mit der gegenüberliegenden Fläche der Ventrikelwand in Kontakt zu kommen, so dass die Flansche und die durch den Teil des hohlen Körpers und den Abschnitt der röhrenförmigen Membran (16) gebildete Anordnung diese abdecken, die Ventrikelwand durch die Öffnung durchqueren und die Position der Fixierungsvorrichtung in der Öffnung blockieren.

2. Vorrichtung nach Anspruch 1, **dadurch gekennzeichnet, dass** das proximale Ende der röhrenförmigen Membran (16) dazu ausgestaltet ist, bei dem Verschieben des Rings (15) fortschreitend deformiert wird, so dass ein Halteflansch (20) gebildet wird, dessen Position in einem vorbestimmten Abstandsbereich ab dem ersten Flansch (18) variieren kann.

3. Vorrichtung nach Anspruch 1 oder 2, **dadurch gekennzeichnet, dass** der Ring (15) an seinem Umfang mindestens einen Koppelfinger aufweist, der dazu bestimmt ist, mit an der Außenfläche (11) des hohlen Körpers platzierten Rasten zusammenzuwirken.

4. Vorrichtung nach Anspruch 1 oder 2, **dadurch gekennzeichnet, dass** die Außenfläche (11) des hohlen Körpers an mindestens einem Teil seiner Außenfläche (11) ein Gewinde umfasst, die Innenfläche des Rings (15) ein Gewinde umfasst, das dazu ausgestaltet ist, mit dem an der Außenfläche (11) des hohlen Körpers platzierten Gewinde zusammenzuwirken, um das Verschieben des Rings (15) zu gestatten.

5. Vorrichtung nach einem der Ansprüche 1 bis 4, **dadurch gekennzeichnet, dass** der Ring (15) einen Rücckehrverhinderungsfinger (21) aufweist, um eine eventuelle Lockerung des Rings (15) nach dem Bilden des Halteflanschs (20) zu verhindern.

6. Vorrichtung nach einem der Ansprüche 1 bis 5, **dadurch gekennzeichnet, dass** die Außenfläche (11) des hohlen Körpers einen Anschlag aufweist, um das Verschieben des Rings (15) zu stoppen.

7. Vorrichtung nach einem der Ansprüche 1 bis 6, **dadurch gekennzeichnet, dass** der Ring (15) und der hohle Körper aus starren und für den menschlichen Körper inerten Materialien ausgeführt sind.

8. Vorrichtung nach einem der Ansprüche 1 bis 7, **dadurch gekennzeichnet, dass** die röhrenförmige Membran (16) aus Nitinol, einer Nitinollegierung oder auch aus expandierbarem Polyurethan ausgebildet ist.

9. Vorrichtung nach einem der Ansprüche 1 bis 8, **dadurch gekennzeichnet, dass** sie dazu ausgestaltet ist, einen Halteflansch (20) mit einem äußeren Durchmesser von streng größer 3/2 D zu bilden, wobei D der Durchmesser der Öffnung in der Ventrikelwand ist.

10. Vorrichtung nach einem der Ansprüche 1 bis 9, **dadurch gekennzeichnet, dass** die Innenwand (12) des hohlen Körpers einen Kanal begrenzt, der sich zwischen dem ersten und dem zweiten Ende des hohlen Körpers erstreckt, die Innenwand (12) Mittel zum dichten Montieren des Körpers einer Herzpumpe an der Wand aufweist, wobei der Pumpenkörper somit mindestens teilweise in dem Kanal aufgenommen ist.

11. Vorrichtung nach Anspruch 10, **dadurch gekennzeichnet, dass** der Körper der Pumpe, die zur Platzierung in dem Kanal bestimmt ist, der von der Innenwand (12) des hohlen Körpers begrenzt ist, ein Gewinde auf seiner Außenfläche (11) aufweist und diese Innenwand (12) ein Gewinde umfasst, das dazu bestimmt ist, mit dem Gewinde des Pumpenkörpers zusammenzuwirken.

12. Herzunterstützungseinheit, umfassend eine Herzantriebspumpe und eine Fixierungsvorrichtung nach einem der Ansprüche 1 bis 11.

## Claims

1. Device for securing on an opening in a ventricular wall, comprising
- a hollow main body with a generally cylindrical form with an outer surface (11),
- a ring (15) which is received at a first end of said hollow body, this ring (15) being mobile along at least part of the outer surface (11) of the hollow body,
- a tubular membrane (16) covering the outer surface (11) of the hollow body, whilst extending between said ring (15) and the end opposite the first end of the hollow body, known as the second end,
- a distal end (17) of this tubular membrane (16), placed on the side of said second end of the hollow body, being self-expandable between a first configuration in which it has a tubular form, and a second configuration in which it defines a first flange (18) extending radially, or substantially radially, starting from said hollow body, this first flange (18) being designed to abut a face of said ventricular wall,
- the other end, known as the proximal end, of this tubular membrane (16) being able to be deformed by the displacement of said ring (15) along the outer surface (11) of the hollow body, such as to form a retention flange (20), the position of which can vary relative to said first flange (18), so as to adapt to ventricular walls with different thicknesses,
- said retention flange (20) being designed to come into contact with the opposite face of said ventricular wall, such that said flanges and the assembly formed by the part of the hollow body and the portion of the tubular membrane (16) covering it, passing through said ventricular wall through said opening, lock said securing device in position in said opening.

2. Device according to Claim 1, **characterized in that** the proximal end of the tubular membrane (16) is configured to be deformed progressively during the displacement of the ring (15), such as to form a retention flange (20), the position of which can vary within a predetermined range of distances starting from the first flange (18).

3. Device according to Claim 1 or 2, **characterized in that** said ring (15) comprises on its periphery at least one coupling finger which is designed to cooperate with notches placed on the outer surface (11) of said hollow body.

4. Device according to Claim 1 or 2, **characterized in that**, with the outer surface (11) of said hollow body comprising a thread on at least part of its outer surface (11), the inner surface of said ring (15) comprises a thread which is configured to cooperate with said thread placed on the outer surface (11) of the hollow body, in order to permit the displacement of said ring (15).

5. Device according to any one of Claims 1 to 4, **characterized in that** said ring (15) comprises a non-return finger (21) in order to prevent any loosening of the ring (15) after formation of the retention flange (20).

6. Device according to any one of Claims 1 to 5, **characterized in that** the outer surface (11) of the hollow body comprises a stop in order to prevent the displacement of said ring (15).

7. Device according to any one of Claims 1 to 6, **characterized in that** said ring (15) and said hollow body are made of rigid materials which are inert for the human body.

8. Device according to any one of Claims 1 to 7, **characterized in that** said tubular membrane (16) is made of nitinol, from a nitinol alloy, or also of expandable polyurethane.

9. Device according to any one of Claims 1 to 8, **characterized in that** it is configured to form a retention flange (20) with an outer diameter which is strictly larger than 3/2 D, where D is the diameter of the opening in the ventricular wall.

10. Device according to any one of Claims 1 to 9, **characterized in that**, with the inner wall (12) of the hollow body delimiting a channel extending between the first and second ends of the hollow body, said inner wall (12) comprises means for sealed assembly of the body of a heart pump and said wall, said pump body thus being received at least partly in said channel.

11. Device according to Claim 10, **characterized in that**, with the body of the pump which is designed to be accommodated in the channel delimited by the inner wall (12) of the hollow body comprising a thread on its outer surface (11), this inner wall (12) comprises a thread which is designed to cooperate with the thread of the pump body.

12. Ventricular assistance unit comprising a propulsive heart pump and a securing device according to one of Claims 1 to 11.
